# EUROPEAN PATENT APPLICATION

(11) **EP 4 667 560 A1**
(43) Date of publication of application: **24.12.2025**
(21) Application number: 24182456.4
(22) Date of filing: 17.06.2024
(51) Int. Cl.: C12N 1/20, A23L 33/135

(54) **NOVEL GABA-PRODUCING LEVILACTOBACILLUS BREVIS STRAIN AND ITS USE**

(71) Applicant: Evonik Operations GmbH, 45128 Essen (DE)
(72) Inventor: VOGEL, Marie-Luise, 33659, Bielefeld (DE); BRAUNER, Aileen Silvana, 33790 Halle/Westfalen (DE); SPECKMANN, Bodo, 63796 Kahl (DE); EHRING, Ellen, 48308 Senden (DE); BRANDT, David, 33659, Bielefeld (DE); TOM DIECK, Heike, 61381 Friedrichsdorf (DE)
(74) Representative: Evonik Patent Association

(57) **Abstract**

The present invention concerns a novel *Levilactobacillus brevis* strain with high capacity to produce gamma-amino butyric acid (GABA) under the conditions occurring in the gastrointestinal tract and preparations thereof as well as compositions and products containing the strain or preparations thereof and further the use of the strain and preparations thereof in food, feed, pharmaceutical and cosmetical compositions as well as in personal care products.

## Description

The present invention concerns a novel *Levilactobacillus brevis* strain with high capacity to produce gamma-amino butyric acid (GABA) under the conditions occurring in the gastrointestinal tract and preparations thereof as well as compositions and products containing the strain or preparations thereof and further the use of the strain and preparations thereof in food, feed, pharmaceutical and cosmetical compositions as well as in personal care products.

Gamma-amino butyric acid (GABA) is an inhibitory neurotransmitter with crucial importance for various functions of the central nervous system, such as modulation of mood, anxiety, stress, pain perception, motor control, and sleep. Further, GABA exerts important peripheral functions in the gut, kidney, vascular system, and skin. Conclusively, microbially produced GABA is a relevant target for probiotic, prebiotic, and synbiotic interventions as well as for the production and use of GABA-enriched ferments. The administration of GABA-producing strains enables a continuous supply of GABA through its production in vivo, which is advantageous compared to the direct application of GABA comprised in oral dosage forms like dietary supplements, foodstuffs, or pharmaceutical products, as the direct application of a high amount of GABA may cause an overdosing effect, in particular irritation of the mucosa. Further, direct oral intake of GABA is problematic due to the unpleasant taste of GABA.

The biosynthesis of GABA is catalyzed by the enzyme glutamic acid decarboxylase (GAD; EC 4.1.1.15) and the cofactor pyridoxal-5-phosphate (PLP) from L-glutamic acid. GAD is widely distributed in both eukaryotes and prokaryotes. The production of GABA is a common property among lactic acid bacteria, especially of genera of the family Lactobacillaceae and of the genus Bifidobacterium. However, this ability is very strain-specific, modulated by regulatory genomic sequences beyond the GAD gene, and typically limited to specific growth conditions (medium composition, temperature, pH, availability and usage of peptides or amino acids). As the biosynthesis of GABA from glutamic acid consumes protons, consequently the GAD genes are normally induced upon low pH to maintain cellular homeostasis under acidic conditions.

According to the state of the art, the ability of microbial strains to produce GABA under conditions occurring in the gastrointestinal tract is limited to low yields and compromised by a low survivability of most strains under such conditions. Therefore, there is a need to provide new strains that can produce high yields of GABA at a broad pH range and further survive the harsh conditions of the gastrointestinal tract.

The ability to produce GABA has already been disclosed in the state of the art for strains of different bacterial taxa. Amongst them, in particular strains of the species *Levilactobacillus brevis* have already been described as potential GABA producers (Lyu et al. 2018; Monteagudo-Mera at al. 2023; Gong, Ren, and Xu 2019; Banarjee et al. 2021; WO 2013/107913).

But for example also bacteria of the species *Lactobacillus plantarum* and *Lactococcus lactis* are disclosed as GABA producers (EP 2173855). Further in particular bacteria of the genus Bifidobacterium have also been disclosed as GABA producers (WO 2013/107913).

*Levilactobacillus brevis* LB01 (CGMCC 16921) is described to convert up to 48 % of monosodium glutamate (1 % w/v in De Man, Rogosa and Sharpe-medium (MRS)) in pH-controlled (pH 5.6 and 6.7) anaerobic batch culture to GABA, yielding a maximal concentration of 28.32 mM after 48 hours (Monteagudo-Mera et al. 2023). Conversion rates of other strains of the species *Lactiplantibacillus plantarum, Limosilactobacillus reuteri, Bifidobacterium adolescentis* or *Bifidobacterium infantis* are reported to be lower. When the CGMCC 16921 strain was cultured in the presence of a complex fecal microbiota, GABA production occurred at pH 5.4-5.6 -but not at pH 6.7-6.9- at conversion rates of up to 68%.

*Levilactobacillus brevis* ATCC 14869 (DSM20054) is reported to grow rather slowly and also to produce not so much GABA)(Banerjee et al. 2021).

On the other hand, the strain *Levilactobacillus brevis* ATCC 367 is reported to have a conversion rate of monosodium glutamate into GABA of almost 100 % (Gong, Ren, an Xu 2019).

Nevertheless there was a need to provide new GABA producing strains which ideally have beneficial characteristics in comparison to the GABA producing strains as already disclosed in the state of the art. In particular there was a need to provide strains which overcome the problems with respect to low survivability under the conditions of the gastrointestinal tract as disclosed before.

As *Levilactobaillus brevis* ATCC 367 was reported to have a conversion rate of monosodium glutamate into GABA of almost 100 %, this strain was used as benchmark strain in the working examples besides the *Levilactobacillus brevis* strain ATCC 14869 (DSM 20054), which often has been used as benchmark strain in the state of the art before.

The object of the invention could be achieved by the identification and isolation of the novel bacterial GABA-producing strain *Levilactobacillus brevis* DSM 34915 derived from South Tyrolean raw milk in 2018. The strain has beneficial and, in their combination, unique properties. Above all, it has exceptionally high capacity to produce the metabolite gamma-aminobutyric acid (GABA), being able to convert L-glutamic acid nearly quantitatively to GABA. Further, it shows a very good survival under low to neutral pH, in the presence of bile, trypsin, and pepsin, i.e. under conditions which occur in the stomach or small and large intestine, respectively.

Thus, a first subject of the present invention are microorganisms and microbial preparations selected from the following group:
a) The strain *Levilactobacillus brevis* DSM 34915;
b) A mutant of the strain *Levilactibacillus brevis* DSM 34915 with a sequence identity of at least 95 %, preferably of at least 97, 98 or 99 %, more preferably of at least 99.5, 99.8 or 99.9 %, in particular of at least 99.95, 99.98 or 99.99 %, to the genomic sequence of the strain *Levilactobacillus brevis* DSM 34915;
c) A preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be a cell-free preparation or may contain living or dead cells or cell debris of such a microorganism or any combination thereof.

The microorganisms of the invention are preferably characterized by at least one, in particular by at least two, three or four, more preferably by all, of the following further features:
- Ability to grow in presence of 2 mM of bile, preferably in presence of 4 mM of bile;
- Ability to survive exposure to a pH of 2.5 for at least 2 hours with a survival rate of preferably at least 50 %, more preferably at least 70 %;
- Ability to survive the exposure to 0.1 mM pepsin at 37°C for at least 4 hours with a survival rate of preferably at least 50 %, more preferably at least 70 %;
- Ability to convert at least 95 %, preferably at least 98 %, more preferably at least 99 %, of the L-glutamate contained in an MRS medium supplemented with 1 wt.-% of monosodium glutamate into GABA at an initial pH value of 6.7 within 48 hours in a batch cultivation;
- Ability to convert at least 50 % of the L-glutamate contained in an MRS medium supplemented with 5 wt.-% of monosodium glutamate into GABA at an initial pH value of 4.5, 5, 5.5, 6 and 6.7 within 48 hours in a batch cultivation;
- Ability to convert monosodium glutamate into GABA in a fermentation medium which contains at least up to 3 wt.-%, preferably at least up to 5 wt.-% monosodium glutamate (295,7 mM) in a batch cultivation;
- Ability to produce at least 15 g/L, preferably at least 20 g/L GABA from 5 wt.-% monosodium glutamate within 48 h at pH 4.5, in particular in MRS medium, in a batch cultivation;
- Ability to efficiently convert L-glutamate (monosodium glutamate) into GABA at a wide pH range, in particular at a pH range from 4.5 to7.2;
- Ability to survive the conditions of the gastric fluid (125 mmol/L NaCl, 7 mmol/L KCI, 45 mmol/L NaHCOs, 3 g/L pepsin, pH adjusted with HCl to 2) at 37°C for at least 4 hours with a survival rate of preferably at least 50 %, more preferably at least 70 %;
- Ability to survive the conditions of the intestinal fluid (0.1 wt.-% pancreatin, 0.15 wt.-% oxgall bile salts in 1 × PBS adjusted to pH 8 with 5 mol/L NaOH) at 37°C for at least 4 hours with a survival rate of preferably at least 50 %, more preferably at least 70 %;
- Ability to survive the conditions of a simulated-colonic-environment (6.25 g/L Bacto tryptone, 2.6 g/L D-glucose, 0.88 g/L NaCl, 0.43 g/L KH₂PO₄, 1.7 g/L NaHCOs, 2.7 g/L KHCOs and 4.0 g/L bile salts) at 37°C for at least 4 hours, with a survival rate of preferably at least 50 %, more preferably at least 70 %.

The microorganisms and microbial preparations according to the invention enhance human and animal health by their exceptional ability to produce GABA and making it available to the body either upon formation *in vivo* or upon administration of *in vitro* produced GABA.

The microorganisms and microbial preparations of the invention can be used in different ways, in particular in food and feed compositions, in particular in functional foods, pet foods, nutraceuticals and foods for special medical purposes (FSMP), in food and feed additives, in food and feed supplements, in pharmaceutical compositions, in cosmetical compositions, in particular for topical use, as well as in personal care products, which all are accordingly also subject matter of the invention. The functional food can in particular be a milk product like milk, cheese, yogurt or curd.

The application of the microorganisms and microbial preparations of the invention can accordingly occur in particular by oral application or topical application, wherein "topical application" in particular also comprises the application to the vaginal skin and/or mucosa.

Thus, a further subject of the invention is also a composition, in particular as mentioned before, containing a microorganism or microbial preparation according to the invention, in particular at least one microorganism or microbial preparation according to the invention, preferably selected from the following group:
a) The strain *Levilactobacillus brevis* DSM 34915;
b) A mutant of the strain *Levilactibacillus brevis* DSM 34915 with a sequence identity of at least 95 %, preferably at least 97, 98 or 99 %, more preferably of at least 99.5, 99.8 or 99.9 %, in particular of at least 99.95, 99.98 or 99.99 %, to the genomic sequence of the strain *Levilactobacillus brevis* DSM 34915;
c) A preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be a cell-free preparation or may contain living or dead cells or cell debris of such a microorganism or any combination thereof.

Thus, a further subject of the invention is in particular also a pharmaceutical composition, containing a pharmaceutically acceptable carrier and a microorganism or microbial preparation according to the invention, in particular at least one microorganism or microbial preparation according to the invention, preferably selected from the following group:
a) The strain *Levilactobacillus brevis* DSM 34915;
b) A mutant of the strain *Levilactibacillus brevis* DSM 34915 with a sequence identity of at least 95 %, preferably at least 97, 98 or 99 %, more preferably of at least 99.5, 99.8 or 99.9 %, in particular of at least 99.95, 99.98 or 99.99 %, to the genomic sequence of the strain *Levilactobacillus brevis* DSM 34915;
c) A preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be a cell-free preparation or may contain living or dead cells or cell debris of such a microorganism or any combination thereof.

A further subject of the invention is therefore also a composition, in particular pharmaceutical composition, in particular as mentioned before, for use in the treatment or prevention of mental, mood and/or sleep disorders or diseases, in particular of pain, anxiety, stress and/or loss of motor control, and/or of disorders or diseases of the gastrointestinal tract, kidney, skin, brain or vascular system, in particular hypertension or high heart rate.

A further subject of the invention is therefore also the use of a microorganism or microbial preparation or of a composition of the invention in the manufacture of a pharmaceutical composition, in particular for treating or preventing mental, mood and/or sleep disorders or diseases, in particular pain, anxiety, stress and/or loss of motor control, and/or disorders or diseases of the gastrointestinal tract, kidney, skin, brain or vascular system, in particular hypertension or high heart rate.

A further subject of the invention is therefore also the non-medical use of a microorganism or microbial preparation or of a composition or of a product of the invention for alleviating or preventing mental, mood and/or sleep disorders or disbalances, in particular anxiety and/or stress.

A further subject of the invention is accordingly also a product, in particular a personal care product, containing a microorganism or microbial preparation according to the invention, in particular at least one microorganism or microbial preparation according to the invention, preferably selected from the following group:
a) The strain *Levilactobacillus brevis* DSM 34915;
b) A mutant of the strain *Levilactibacillus brevis* DSM 34915 with a sequence identity of at least 95 %, preferably at least 97, 98 or 99 %, more preferably of at least 99.5, 99.8 or 99.9 % in particular of at least 99.95, 99.98 or 99.99 %, to the genomic sequence of the strain *Levilactobacillus brevis* DSM 34915;
c) A preparation of a microorganism according to (a) or (b), wherein the preparation is preferably an optionally concentrated or dried fermentation broth, supernatant of a fermentation broth, ferment, cell lysate or cell extract of a microorganism according to (a) or (b), where the preparation may be a cell-free or may contain living or dead cells or cell debris of such a microorganism or any combination thereof.

A further subject of the invention is accordingly also the use of a microorganism of the invention for producing GABA and/or a GABA containing product, preferably by cultivating a microorganism of the invention in a suitable fermenter and preferably by using a suitable fermentation medium containing glutamic acid or a salt thereof or at least one source for glutamic acid or of a salt thereof, wherein the source of glutamic acid or salt thereof is preferably selected from peptides, protein hydrolysates, Spirulina powder, bacterial extracts, gamma-polyglutamate and Natto powder, and wherein after the fermentation is ended the GABA containing supernatant is preferably separated mechanically from the biomass to obtain a GABA containing product, which may be concentrated and/or dried subsequently.

A further subject of the invention is accordingly also a method of producing GABA and/or a GABA-containing product, wherein a microorganism according to the invention is employed, wherein the microorganism is preferably cultivated in a medium which contains glutamic acid or a salt thereof or at least one source for glutamic acid or of a salt thereof, wherein the source of glutamic acid or salt thereof is preferably selected from peptides, protein hydrolysates, Spirulina powder, bacterial extracts, gamma-polyglutamate and Natto powder, and wherein after the fermentation is ended the GABA containing supernatant is preferably separated mechanically from the biomass to obtain a GABA containing product, which may be concentrated and/or dried subsequently.

The strain *Levilactobacillus brevis* DSM 34915 was identified by screening of naturally occurring isolates and was deposited at the DSMZ (Leibniz-lnstitute DSMZ-German Collection of Microorganisms and Cell Cultures, Inhoffenstraße 7B, 38124 Braunschweig, Germany) on January 23, 2024 under the provisions of the Budapest Treaty on the International Recognition of the Deposit of Microorganisms for the Purpose of Patent Procedure in the name of the applicant Evonik Operations GmbH. Originally, the strain was isolated from South Tyrolean food.

The *Levilactobacillus brevis* strain DSM 34915 exhibits the following characterizing sequences: a 16S rDNA sequence according to SEQ ID NO: 1, an rpoB sequence according to SEQ ID NO: 2, a gyrB sequence according to SEQ ID NO: 3, an ftsY sequence according to SEQ ID NO: 4, an ychF sequence according to SEQ ID NO: 5 and a sequence according to SEQ ID NO: 6 which is located on the site G1487_01825 and codes for a DNA-binding response regulator.

Accordingly, the mutants of the strain DSM 34915 preferably exhibit a 16S rDNA sequence, an rpoB sequence, a gyrB sequence, a ftsY sequence, an ychF sequence and/or said sequence located on the site G1487_01825 coding for a DNA-binding response regulator, which are at least 99 or 99.5 %, more preferably at least 99.8 or 99.9 %, above all 100 % identical to the respective sequences of the strain DSM 34915 as mentioned above.

The mutants of the strain DSM 34915 have preferably further a sequence identity of at least 99 %, more preferably of at least 99.5 %, in particular of at least 99.8 or 99.9 % to the genomic DNA of the strain DSM 34915.

The mutants of the strain DSM 34915 further preferably comprise a gad operon which has a sequence identity to the gad operon of the strain DSM 34915 of at least 99.5 %, more preferably of at least 99.8 % or 99.9 %, in particular of 100 %.

The mutants of the invention can principally be obtained by any kind of method, i.e., by GMO- or non-GMO methods, but preferably the mutants are not genetically modified, i.e., non-GMO. This means that the mutants of the deposited strains are preferably either also naturally occurring microorganisms or spontaneous mutants of such naturally occurring microorganisms, in particular of the deposited strains, or microorganisms which are obtained by another method which is classified as non-GMO. The term "spontaneous mutant" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible.

Spontaneous mutants might further, but less preferably, be obtained by using mutagens, i.e., chemical substances which induce the formation of mutants. As formation of spontaneous mutants by using mutagens is less preferred, in a preferred embodiment of the invention the spontaneous mutants and/or non-GMO mutants are obtained without the use of such mutagens. If the mutants are obtained by applying gene technological and/or biotechnological methods like site-directed mutagenesis, then preferably methods are applied which are classified as non-GMO. A non-GMO method according to the invention is preferably characterized in that the method does not involve introduction of heterologous genetic information into the microorganism. In a particularly preferred embodiment of the invention the microorganisms of the invention are naturally non-occurring mutants, in particular non-GMO and/or spontaneous mutants as defined before. The mutants of the invention have preferably the same characteristics like the parent strain from which they are derived.

The cells of *Levilactobacillus brevis* are preferably present, in particular in the preparations and compositions of the invention, as living cells, i.e., in the vegetative state. But besides that the cells can also be used in the non-living, inactivated form, or as a combination of living and inactivated cells, as also the inactivated cells are expected to still have a certain probiotic effect.

Accordingly, preparations of the microorganisms of the invention, which are a preferred subject of the invention, may be preparations containing intact cells, inactivated cells, cell debris or mixtures thereof. Further, the preparations may also be cell-free preparations, wherein the cell-free preparations may contain cell debris or may be free of cell debris. Particularly preferred examples for preparations of the microorganisms of the invention are the fermentation broth, as obtained after finishing the fermentation of the cells, the supernatant of the fermentation broth, which is obtained by separating all or the major part of the cells from the fermentation broth, as well as cell lysates and cell extracts, i.e. cytosol preparations, which can be obtained by breaking the microbial cells. Such preparations may also be used in concentrated or dried form, wherein the dried form has preferably a total dry matter content of at least 90 wt.-%, more preferably of at least 95 wt.-%.

The term "fermentation broth" according to the invention refers to the product of a cultivation of bacteria in a suitable fermentation medium. Methods for producing such fermentation broths are well known to those skilled in the art. The fermentation broths of the present invention can for example be obtained by culturing the strains by using the media, conditions and methods as described in US 6,060,051, EP 0287699, US 2014/0010792 or in the FAO Report 179 (2016): "Probiotics in Animal Nutrition". Conventional large-scale microbial culture processes include submerged fermentation, solid state fermentation, or liquid surface culture. Typically, fermentation is carried out, until a certain cell density is reached like an optical density (OD 600) of about 5 to 25, more preferably 10 to 22, above all 15 to 20. The fermentation broths preferably contain cells in an amount of 1×10³ to 1×10¹³ CFU, more preferably in an amount of 1×10⁵ to 1×10¹² CFU, above all in an amount of 1×10⁷ to 1×10¹¹ CFU per ml of fermentation broth.

The term "fermentation broth" according to the invention refers to the direct product of the cultivation, i.e. a suspension preferably containing cells in the amount as mentioned in the previous paragraph, as well as to concentrated and dried forms of such a fermentation broth, wherein the dried form ("dried fermentation broth") preferably has a total dry matter content of at least 95 wt.-%.

Drying of the preparations of the invention can be carried out by applying methods as generally known to those skilled in the art, in particular by evaporation of water, freeze-drying, lyophilization, spray drying, spray granulation, fluidized bed drying, vacuum drying, tray drying, drum drying and combinations thereof. After drying, the dried preparations as obtained can be further worked up, in particular by grinding and/or granulation.

Cell-free preparations of the microorganisms of the invention can be obtained by centrifugation, filtration, in particular ultrafiltration or microfiltration, decantation and/or flotation of the fermentation broth or of a suspension as obtained after breaking the microbial cells. Depending on the technique used, these cell-free preparations may not be completely devoid of cells, but may still comprise a smaller amount of cells, in particular up to 20 wt.-% or up to 10 wt.-% of cells. As the cells produce and secret compounds like metabolites, enzymes and/or peptides into the surrounding medium, the supernatants, extracts and lysates of the cells comprises a mixture of such compounds, in particular metabolites, enzymes and/or peptides, as secreted and/or produced by the cells.

Cell lysates can be prepared either directly by breaking the cells of an optionally dried fermentation broth or by breaking the cells after first separating the cells from the fermentation broth mechanically, in particular by filtration, centrifugation and/or decantation.

Breaking of the cells can be carried out by applying techniques as known to those of skill in the art, in particular mechanically, chemically and/or enzymatically. Depending on the degree of force applied, a composition comprising only ruptured cells or a composition comprising a mixture of cell debris and intact cells is obtained. Homogenization of the cells may be realized for example by applying high pressure, high pH, osmotic shock, heat shock, sonication, homogenization or shearing, in particular by utilizing means selected from French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer.

After breaking of the cells, the cell debris and remaining cells, if any, can optionally be separated from the cytosol preparation thus obtained to obtain a cell extract, i.e. a cytosol preparation, which is free of cells and cell debris.

The fermentation broths of the invention, from which the cells have been removed, i.e. the cell-free supernatant and metabolites containing fractions of the fermentation broth, are also denoted as "postbiotics", "metabiotics" or "biogenics".

A further specific preparation of the invention is a preparation containing inactivated cells, i.e. cells which are not able to grow anymore. Preparations containing inactivated cells are also known as "paraprobiotics". Such preparations can be obtained for example by heat and/or pH inactivation of the cells, preferably by heat and/or pH inactivation of a fermentation broth containing the cells.

Inactivation of the cells can be carried out, e.g., by heat treatment and/or by adjusting an acid or an alkaline pH. Heat inactivation of the cells is preferably carried out by increasing the temperature to between 60°C and 80°C and incubation for at least 30 minutes. pH inactivation at acidic pH is preferably carried out by lowering the pH to at least 3 by addition of an acid like 5M H₂SO₄ and incubation for at least 30 minutes, preferably at least one hour. pH inactivation at alkaline pH is preferably carried out by increasing the pH to at least 10 and incubation for at least 30 minutes, preferably at least one hour. Alternatively, inactivation of the cells may also be carried out by applying gamma- or UV-irradiation. In one specific embodiment of the invention a fermentation broth is used, in which at least 90 %, more preferably at least 95 or 99 %, in particular all bacterial cells are inactivated. Correspondingly, in this embodiment of the invention a fermentation broth is used which preferably contains no viable cells, at all. Such a product, which contains mainly or exclusively inactivated cells, is preferably used in cosmetical and/or topical applications and is called "ferment" in the context of the present invention.

The preparations of the invention comprise GABA, preferably in a high amount. They preferably also comprise further organic acids, in particular lactate and acetate, preferably in a lower amount. In addition they may comprise metabolites, enzymes and/or peptides as produced by the microorganisms of the invention in smaller amounts. Separation of GABA from cell debris, if necessary, can simply be carried out by centrifugation, decantation or filtration.

Preferably according to the invention always an effective amount of the microorganisms, microbial preparations, in particular fermentation broths, compositions and/or products of the invention is used in the embodiments of the invention.

Compositions and products of the invention preferably contain the microorganisms of the invention in an amount of from 1×10² to 1×10¹² CFU, more preferably in an amount of 1×10³ to 1×10¹⁰ CFU, above all in an amount of 1×10⁴ to 1×10⁹ CFU and/or in an amount of from 0.1 to 20 wt.-%, preferably 0.2 to 15 wt.-%, in particular 0.5 to 10 wt.-%, above all 1 to 5 wt.-%.

In context with food and feed compositions of the invention, the term "effective amount" refers to an amount which effects at least one beneficial effect to the gastrointestinal tract of human beings or animals in comparison to the gastrointestinal tract of human beings or animals that have not been exposed to the microorganisms, microbial preparations and/or compositions of the invention, but may have been exposed to the same treatment and/or to the same food or feed compositions depleted by such microorganisms or microbial preparations.

In context with cosmetical compositions of the invention, the term "effective amount" refers to an amount which effects at least one beneficial effect to the human skin in comparison to a human skin that has not been exposed to the microorganisms, microbial preparations and/or compositions of the invention, but may have been exposed to the same treatment and/or to the same cosmetical composition depleted by microorganisms or microbial preparations.

The microorganisms and microbial preparations of the invention can also be provided in combination with a suitable carrier, wherein the carrier is preferably an inert formulation ingredient added to improve recovery, efficacy, or physical properties and/or to aid in packaging or administration of the microorganisms or microbial preparations. Such carriers may be used individually or in combination and can be added either as part of the fermentation medium, in the course of the fermentation or after the fermentation of the microorganisms has been ended.

The carrier is preferably selected from anti-caking agents, antioxidants, bulking agents, binders, structurants, coatings and/or protectants. Examples of useful carriers include polysaccharides (in particular starches, maltodextrins, celluloses, methylcelluloses, gums like guar gum, xanthan gum and gum arabic, wheat middlings, corn cob meal, chitosan and/or inulins), protein sources (in particular skim milk powder, sweet-whey powder, gelatine and/or soy flour), protein hydrolysates (in particular gelatine, yeast extract and/or peptones like soy peptone), peptides, sugars (in particular lactose, trehalose, sucrose, dextrose and/or maltose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), silicates (in particular clays, zeolites, Fuller's earth, clintpolite, montmorillonite, perlite, vermiculite, diatomaceous earth, talc, bentonites, kaolin clay, silica in particular precipitated silica, hydrophobic silica and/or hydrophilic silica, and/or silicate salts like aluminium, magnesium and/or calcium silicate), silica gel, silica dioxide, activated carbon, lignite, magnesium and calicum oxide.

Compositions and products of the invention preferably contain glutamic acid or a salt thereof or at least one source of glutamic acid or of a salt thereof (referred to as "glutamate source"). The glutamate source is preferably selected from peptides, protein hydrolysates, Spirulina powder, bacterial extracts, in particular extracts of Bacillus and Corynebacteria, gamma-polyglutamate and Natto powder. Instead of a direct source of glutamate/glutamic acid also a source can be used, which can easily be converted enzymatically either by the microorganism of the invention or by the human or animal body into glutamate/glutamic acid. Such sources are glutamine and salts thereof like glutamine hydrochloride, glutamine containing peptides like Ala-Gln and for example folic acid.

The glutamic acid or salt thereof or the source of glutamic acid or salt thereof is, if present, preferably contained in the compositions and products of the invention in an amount of from 0.1 to 50 wt.-%, preferably in an amount of from 0.2 to 40 wt.-% or 0.5 to 30 wt.-%, in particular in an amount of from 1 to 20 wt.-%, 1.5 to 15 wt.-%, 2 to 12 wt.-% or 2.5 to 10 wt.-%. In a very preferred embodiment, the compositions and products of the invention contain glutamic acid or a salt thereof in an amount of from 0.1 to 25 wt.-%, preferably in an amount of from 0.5 to 20 wt.-%, in particular in an amount of from 1 to 10 wt.-%.

Compositions of the invention, in particular as mentioned before, preferably contain at least one further additive selected from carriers, in particular as mentioned before, further probiotics, prebiotics, enzymes, vitamins, minerals, further amino acids, peptides, plant extracts and algal extracts.

The at least one further probiotic microorganism is preferably contained in the compositions of the invention, if present, in an amount of about 1×10² to about 1×10¹⁰ CFU/g, more preferably in an amount of about 1×10⁴ to about 1×10⁹ CFU/g, in particular in an amount of 1×10⁶ to 1×10⁸ CFU/g. It is used preferably in form of an optionally dried fermentation broth.

Such further probiotic microorganisms are preferably bacteria selected from the genera Bacillus, Lactobacillus, Lacticaseibacillus, Lactiplantibacillus, Levilactobacillus, Bifidobacterium, Enterococcus or Pediococcus. Probiotic microorganisms of the genus Bacillus are preferably selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Bacillus nakamurai* and *Bacillus siamensis.* Probiotic bacteria of the genus Enterococcus are preferably of the species *Enterococcus faecium.*

Probiotic bacteria of the genus Pediococcus are preferably of the species *Pediococcus acidilactici.* Further suitable probiotic microorganisms are E. *coli* Nissle and *Clostridium butyricum.*

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used according to the invention and which may aid in the digestion of feed, are preferably selected from phytases (EC 3.1 .3.8 or 3.1 .3.26), xylanases (EC 3.2.1 .8), galactanases (EC 3.2.1 .89), galactosidases, in particular alpha-galactosidases (EC 3.2.1.22) and beta-galactosidases, proteases (EC 3.4), lipases, phospholipases, in particular phospholipases A1 (EC 3.1 .1 .32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ), lysozymes (EC 3.2.1 .17), glucanases, in particular beta- glucanases (EC 3.2.1 .4 or EC 3.2.1 .6), glucoamylases, cellulases, pectinases, glutamate decarboxylases, or any mixture thereof.

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof. Amino acids which may be used according to the invention are for example lysine, alanine, threonine, methionine or tryptophan, or combinations thereof.

Further amino acids which may be used according to the invention are in particular proteinogenic amino acids as known to those skilled in the art, in particular L-threonine, L-methionine, L-valine, L-lysine and L-tryptophan.

Dipeptides which may be used according to the invention are in particular any dipeptides of proteinogenic amino acids.

Plant extracts are according to the invention preferably selected from extracts from broccoli, olive fruit, pomegranate, blackcurrant, blueberry, bilberry, sea buckthorn, camu camu, boysenberry, curcuma, ginger, garlic, grape seeds, acai berry, aronia, goji berry, horseradish, rice, beans, kudzu, bamboo, hokkaido, boswellia serrata, spirulina, panax ginseng, cannabidiol, rose hip, pu erh, sea grass, sencha, echinacea and green tea leaves.

Compositions of the invention, in particular feed, food and pharmaceutical compositions of the invention, preferably comprise at least one further feed or food additive.

Suitable typical feed or food additives which may be contained in the feed, food and pharmaceutical compositions according to the invention include one or more of the following: proteins, carbohydrates, fats, feed concentrates, silage, mashfeed, probiotics, prebiotics, enzymes, vitamins, immune modulators, milk replacers, minerals, amino acids, carriers, in particular as mentioned above, coccidiostats, acid-based products and/or medicines, in particular antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

Immune modulators which may be used are for example antibodies, cytokines, spray-dried plasma, interleukins, or interferons, or combinations thereof.

The compositions of the invention may further comprise betaine and/or choline and/or other physiologically effective methyl group donors. The compositions may further comprise polyunsaturated fatty acids, in particular DHA and/or EPA.

Thus, a further embodiment of the invention is also a method of preparing a feed, food or pharmaceutical composition comprising mixing a compound or composition of the invention, in particular in an amount effective to enhance animal health, in particular gut health, with feed or food ingredients, such as proteins, lipids and/or carbohydrates, and optionally further beneficial substances, preferably as mentioned before, to provide a feed, food or pharmaceutical product. This method may comprise also a pelleting and/or extruding step. Thus, in a preferred embodiment of the invention the feed compositions are feed or food pellets and/or feed or food extrudates.

The food composition of the invention can in particular be a functional food, a food for special medical purposes, a nutraceutical or a dietary supplement. The dietary supplement can in particular be a pill, a tablet, a capsule or a liquid.

The compositions of the invention, in particular the food and feed compositions, may also be contained in a capsule, in particular in a bile-resistant capsule.

The compositions of the invention, in particular the food and feed compositions, may also comprise a coating, in particular an enteric coating, wherein the enteric coating preferably comprises one or more of the following substances: methyl acrylate-methacrylic acid copolymers, cellulose acetate phthalate (CAP), cellulose acetate succinate, Hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

A further subject of the invention is also the use of the microorganisms or microbial preparations or feed compositions of the invention for feeding animals, in particular selected from ruminants, swine, poultry and aquaculture animals.

A further subject of the invention is accordingly also a method of feeding animals, in particular selected from ruminants, swine, poultry and aquaculture animals, wherein microorganisms, microbial preparations or feed compositions of the invention are employed.

The cosmetical composition of the invention is preferably a topical skin care composition.

According to the invention the term "skin" refers preferably to the skin itself, particularly to the human skin, but may also refer to the mucosa and skin adnexa, in so far that they include living cells, particularly hair follicles, hair roots, hair bulbs, the ventral epithelial layer of the nail bed (lectulus) as well as sebaceous glands and perspiratory glands.

The cosmetic or pharmaceutical composition according to the invention can be in any form suitable for application on the skin, e.g., a soap, a lotion, a serum, a spray, a jelly, a cream, a gel, a paste, a pomade, a balm, an ointment, a foam, a mousse, an emulsion, a stick, a suppository, a patch, a powder, a cleaning fluid or cleaning milk, a deodorant, an anti-perspirant, a salve, a hair conditioner or a shampoo. The composition can also be applied in a mask or in a band-aid, particularly in a gel reservoir mask or band-aid or matrix mask or band-aid.

The cosmetical compositions of the invention preferably have a pH in the range of between 3 to 8, in particular in the range of 4.0 to 8.0, more preferably in the range of 4.5 to 7.4, particularly preferably in the range of 5.0 to 7.2. The pH of the composition is preferably determined at 22°C after stirring for five minutes using a pH electrode calibrated in accordance with ISO 4319 (1977).

The application area can be the skin of any part of the body, particularly the facial skin, the scalp, the skin on hands and feet, the skin under the arms, as well as the mucous membrane, wherein the facial skin and the mucosa in the vaginal area are particularly preferred.

In a very preferred embodiment of the invention the cosmetical composition is a composition for topical application to the vaginal mucosa and is in particular selected from suppositories, creams and gels.

The cosmetical composition of the invention is preferably an emulsion, in particular a W/O or an O/W emulsion. This means that the composition contains preferably at least one surface-active substance as emulsifier or as dispersion agent. Emulsifiers act at the interphase to produce water or oil-stable adsorption layers that protect the dispersed droplets against coalescence and thereby stabilize the emulsion. Thus, emulsifiers, like surfactants, are composed of hydrophobic and hydrophilic molecular moieties. Hydrophilic emulsifiers preferably form O/W emulsions and hydrophobic emulsifiers preferably form W/O emulsions. An emulsion is understood to mean a dispersion of a liquid in the form of droplets in another liquid using an energy input to afford interphases stabilized with surfactants. The choice of this emulsifying surfactant or emulsifier depends on the materials being dispersed and the respective external phase as well as the fineness of the emulsion.

In addition to or instead of the emulsifiers as mentioned above the cosmetical compositions according to the invention may also comprise foaming, non-ionic, zwitterionic, anionic and/or cationic surfactants and/or biosurfactants as further surface-active ingredients.

The cosmetical or pharmaceutical skin care composition of the invention preferably comprises at least one further ingredient selected from phospholipids, sphingoid bases, free fatty acids, protein hydrolyzates, polymers, light stabilizers, anti-perspirants, deodorants, peptides, amino acids, vitamins, extracts from plants and algae, cosmetic oils, emollients, antioxidants, preservatives, thickeners, viscosity regulators, stabiliziers, hydrotropes, solids, fillers, film formers, conditioners, insect repellents, self-tanning agents, odor absorbers, solvents, perfumes and dyes.

A further subject of the invention is accordingly also the use of the microorganisms, microbial preparations and/or cosmetical compositions of the invention for treating the human skin, in particular the skin and/or mucosa in the vaginal region.

A further subject of the invention is accordingly also a method of treating the human skin topically, in particular the skin and/or mucosa in the vaginal region, wherein the microorganisms, microbial preparations and/or compositions of the invention are employed.

As provision of GABA is also an important topic for female health, thus a further subject of the invention are also personal care articles which contain microorganisms or microbial preparations of the invention, wherein the personal care articles are preferably disposable absorbent articles, in particular selected from sanitary napkins, panty liners, tampons, diapers, incontinent pads, breast pads, perspiration pads, human or animal waste management devices and interlabial pads.

The personal care articles preferably contain a water-absorbing gelling material, wherein the water-absorbing gelling material is preferably a water-absorbing composition comprising water-absorbing polymer structures, wherein the water-absorbing polymer structures are preferably based on partly neutralized, crosslinked polyacrylates. Very preferably, the personal care articles contain superabsorbent polymers.

Superabsorbent polymers (superabsorbers) are water-insoluble crosslinked polymers which are capable of absorbing and retaining under pressure large amounts of aqueous liquids, in particular body fluids, preferably urine or blood, with swelling and formation of hydrogels. In general, these uptakes of liquid are an amount of water at least 10 times or even at least 100 times the dry weight of the superabsorber or superabsorbent compositions. Due to these characteristic properties, these polymers are very suitable to be used in sanitary articles. A comprehensive overview of superabsorbers and superabsorbent compositions, their use and their preparation is given by F. L. Buchholz and A. T. Graham (editors) in "Modern Superabsorbent Polymer Technology", Wiley-VCH, New York, 1998.

Suitable water-absorbing gelling materials and methods of preparing them are described or mentioned for example in WO 2009/040106.

The microorganisms and microbial preparations can further be immobilized on the water-absorbing gelling material with aid of a binder, in particular with aid of an organic or inorganic binder. Suitable binders for this purpose are also disclosed in WO 2009/040106 and are preferably selected from thermoplastic hot melt adhesives and hydrophilic, preferably water-soluble, polymers. Thermoplastic hot melt adhesives which are preferably employed are described more in detail WO 2005/011860. Hydrophilic, preferably water-soluble, polymers which can be employed are in particular polyvinyl alcohols, which can be obtained by partial or complete hydrolysis of polyvinyl acetate, or polyalkylene glycols, in particular polyethylene glycols having a molecular weight in a range of from 1,000 to 50,000 g/mol, particularly preferably in a range of from 1,500 to 25,000 g/mol and most preferably in a range of from 2,000 to 15,000 g/mol.

The personal care articles preferably contain besides at least one microorganism or microbial preparation of the invention at least one source of glutamate, in particular as mentioned before.

### Description of the figures

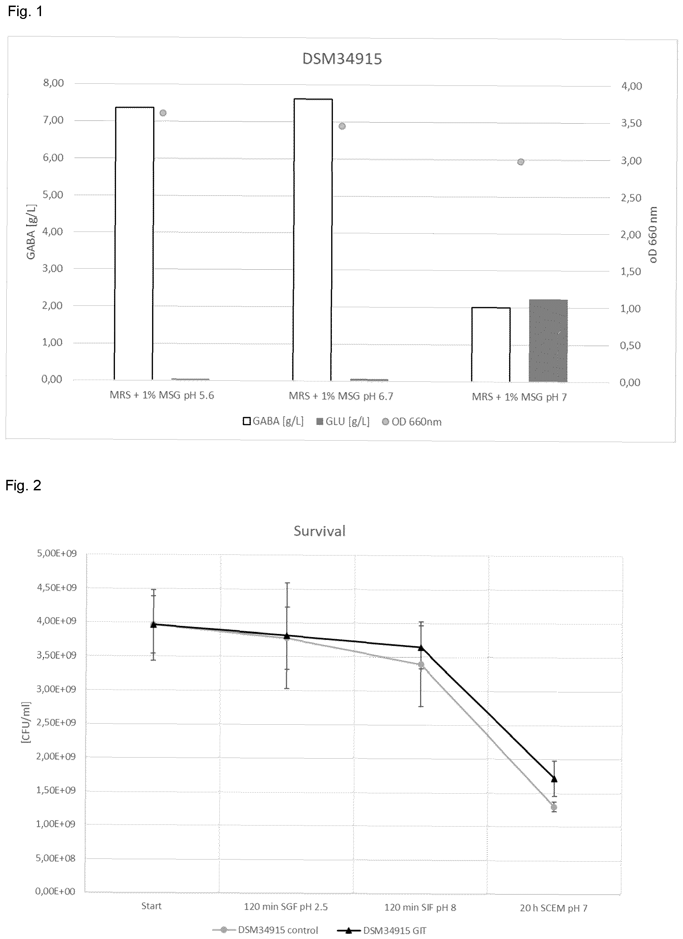

Figure 1 relates to results as obtained according to working example 2 and shows the total GABA concentration [g/L] as produced by the strain DSM34915 in MRS medium supplemented with 1 wt.-% DL-Glu starting from different initial pH levels (pH 5.6, 6.7 or 7.0) after 48 h of cell cultivation (see also Table 3). Depicted are further the residual concentration of DL-Glu [g/L] in the fermentation broth and the optical density [OD] at 660 nm at the end of the fermentation.
Figure 2 relates to the results as obtained according to working example 3 and shows the survival rate of the strain DSM34915 [CFU/ml] tested under gastrointestinal conditions, i.e., incubation for 2 hours in artificial gastric fluid at pH 2, followed by incubation for 2 hours in artificial intestinal fluid at pH 8, and subsequent incubation for 20 hours in simulated-colonic-environment medium (SCEM) at pH 7, compared to the survival rate as obtained at cultivation of the strain in neutral PBS medium for 24 hours (see also Table 4).

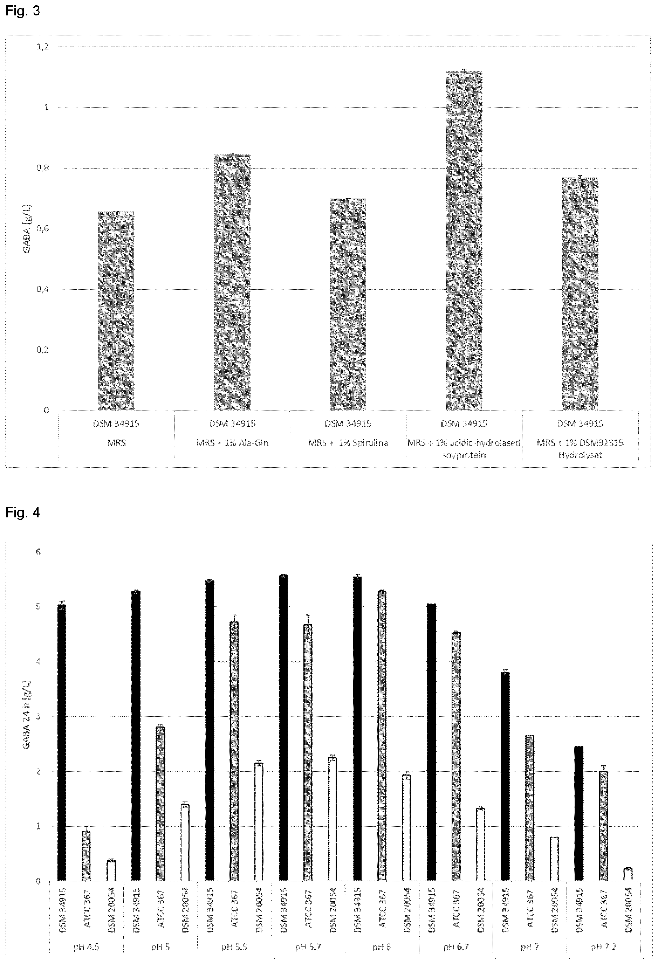

Figure 3 relates to the results as obtained according to working example 4 and shows the results with respect to the use of different D.L-Glu containing substrates for the production of GABA. Strain DSM34915 was cultivated in MRS medium supplemented with 1 wt.-% Ala-Gln, Spirulina extract, acidic hydrolyzed soyprotein or Bacillus subtilis DSM32315 hydrolysate and the resulting GABA concentration was subsequently determined (see also Table 5).
Figure 4 relates to the results as obtained according to working example 5 and depicts the GABA production performance of strain DSM34915 at different initial pH levels in comparison to *Levilactobacillus brevis* benchmark strains ATCC367 and DSM20054. All strains were cultivated in MRS medium supplemented with 1 wt.-% MSG and adjusted to different initial pH levels. The GABA concentration [g/l] was measured after 24 h of cultivation.

### Working examples

### Example 1: Screening of GABA-producing lactic acid bacteria cultured in complex medium

A selection of 41 genomically different lactic acid bacteria strains of the family Lactobacillaceae isolated from 16 different food sources like cheese, sourdough, kefir and raw milk were screened regarding GABA formation in a complex lactic acid bacterium medium without intentional addition of GABA precursors at an initial pH of 5.6. For the screening a single colony of each strain was precultured in 10 ml De Man, Rogosa and Sharpe-medium (MRS) (VWR (84.613.500): 10 g/L enzymatic digest of casein, 10 g/L meat extract, 5 g/L yeast extract, 20 g/L glucose, 2 g/L dipotassium hydrogen phosphate, 5 g/L sodium acetate, 2 g/L di-ammonium citrate, 0.2 g/L magnesium sulphate, 0.05 g/L manganese sulphate, 1.08 g/L polyoxyethylenesorbitan monooleate (Tween 80)) supplemented with 0.05% L-cysteine hydrochloride monohydrate (Sigma-Aldrich, C7880-100G)) at 37°C and anaerobic conditions. After 24 h a fresh culture of 2.5 ml MRS-medium was inoculated with an optical density of 0.1 at 660 nm for at least 24 h at 37°C under anaerobic conditions. The strains were cultivated in 24 deep-well plates and the cultures were harvested by centrifugation (10 min, 4000 rpm) and the cell-free supernatant was subsequently analyzed analytically regarding the content of total glutamic acid (DL-GLU) and GABA. The amount of produced GABA was compared to the control sample without lactic acid bacteria inoculum. For the determination of DL-GLU and GABA a liquid chromatographic separation of amino acids according to a respective standard substance of known concentration is used. The method is validated for the use of several amino acids in a hydrolysate program with a highly acidic ion exchanger. The free amino acids are separated by a post-chromatographic reaction stained with ninhydrin. The resulting dye is produced at a specific wavelength (A= 570 nm / 440 nm) and the absorbance is evaluated with suitable software (e.g. Chromeleon). The chromatographic separation was performed using an Amino Acid Analyzer System S433 from Sykam equipped with a pre-column, 100 x 4.6 mm, 7 µm and separation column, 150 x 4.6 mm, 7 µm with a flow rate of 0.45 mL/min. The mobile phase consists of a mixture of Eluent A (trisodium citrate dihydrate, citric acid, methanol, HCL (37%), octanoic acid, pH 3.45) and Eluent B (trisodium citrate dihydrate, boric acid, octanoic acid, NaOH, pH 10.18) (0%-100%). The sample was prepared by diluting the stock solution in an acid buffer and injecting 50 µl onto the column. The retention time of GLU is 14.183 min and GABA has a retention time of 35.833 min. Calibration curves were constructed using standard solutions, and the peak areas were used for quantification.

As a result of the screening the strain *Levilactobacillus brevis* DSM 34915 was identified to produce GABA under the applied conditions. Highest amounts of GABA were produced in a simple cultivation for 24 h. In total 0.729 g/L GABA could be produced from 0.89 g/L precursor substrate which is determined as DL-glutamic acid (preferred is the L-form). The substrate and the enzymatic cofactor pyridoxal-5-phosphate are part of the complex composition of MRS medium. The strain is able to use all of the substrate to produce GABA which means a 100% conversion under the applied conditions.

**Table 1: GABA amounts produced in a small cultivation in MRS medium, containing DL-Glu in an amount of 0.89 g/L**

| Strain ID | Species | Origin of Isolation | Total GABA [g/L] | Total GABA [mM] |
|---|---|---|---|---|
| DSM34915 | *Levilactobacillus brevis* | South Tyrolean raw milk | 0.729 (+/- 0.025) | 7.06 (+/- 0.242) |

### Example 2: Formation of GABA under different growth conditions

After the identification of *Levilactobacillus brevis* DSM34915 as GABA producer, showing a high production of GABA in MRS medium at an initial pH of 5.6, the strain was tested in MRS medium as described above supplemented with 1 % monosodium glutamate (10 g/L MSG, >99%, Sigma-Aldrich G1626). GABA production was assessed at two different starting pH values (pH 4.5 and 5.5) to mimic conditions of the stomach and proximal colon, respectively. The initial pH was adjusted to 5.5 and 4.5 by using HCl (37%). Prior to the assessment of GABA production, the strain was cultivated on MRS-agar (VWR; 1.5% agar-agar-BioScience, granulated, Roth (1347.2)) for two days at 30°C under anaerobic conditions (90% N₂and 10% CO₂; Oxoid^{™} AnaeroGen^{™} 2.5 L packs - Thermo Scientific, AN0025A). One colony was used, respectively, as inoculum for further cultivation in 10 ml MRS medium supplemented with 0.05% L-cysteine-HCL as mentioned in Example 1. Subsequently, the strain DSM 34915 was inoculated at a concentration of approximately 10⁷ CFU/ml (final OD₆₆₀ₙₘ of 0.1) in a Woulter-Deutz small cultivation system containing 8 ml of prepared MRS-medium supplemented with 1% (w/v; 59 mM) MSG at pH 4.5 or 5.5. Bacterial cultures were incubated for 48 h at 37°C under anaerobic conditions (Anaerobic jar 2.5 L; Oxoid^{™} AnaeroGen^{™} Compact packs - Thermo Scientific, AN0020D). Samples were collected at baseline (0) and after 48 h of cultivation and cell-free supernatants were analyzed using liquid chromatographic separation of amino acids as described in Example 1. The concentration of L-glutamate is given as DL-glutamic acid as both are two forms of the same amino acid. L-glutamate is the ionized form of L-glutamic acid. In aqueous solution, L-glutamic acid is rapidly deprotonated to form L-glutamate. Therefore, L-glutamate is measured as L-glutamic acid analytically and used interchangeably.

By supplementing MRS-medium with 1% MSG (10 g/L; 59 mM) the cells of DSM34915 have a higher substrate availability and are able to synthesize GABA as a reaction of the natural stress response in presence of lower pH conditions. As shown in Table 2, the strain DSM34915 can produce higher amounts of GABA at an initial pH of 5.5 than under an initial pH of 4.5. The highest GABA concentration of 6.406 g/L was produced by *Levilactobacillus brevis* DSM34915 after 48 h in a small cultivation system under anaerobic conditions. Normalized to the optical density at 660 nm, the strain produces 2.046 g/L per OD_{660 nm} at an initial pH of 5.5. DSM34915 is thus able to produce GABA under different pH levels that occur under gastrointestinal conditions.

**Table 2: GABA amounts produced in a small cultivation in MRS medium supplemented with 1% monosodium glutamate, containing DL-Glu in an amount of 8.2 g/L (pH 4.5) or 9.66 g/L (pH 5.5), respectively. Total GABA values in g/L and normalized values related to the final OD measured at 660 nm are depicted.**

| Strain ID | Species | Total GABA [g/L] | Total GABA [g/L] | GABA [g/L]/OD₆₆₀ₙₘ | GABA [g/L]/OD₆₆₀ₙₘ |
|---|---|---|---|---|---|
| | | pH 4.5 | pH 5.5 | pH 4.5 | pH 5.5 |
| DSM34915 | *Levilactobacillus brevis* | 2.691 | 6.406 | 1.293 | 2.046 |

Likewise, the strain DSM 34915 was tested under the same test set-up mentioned above but under different initial pH values. The strain was tested in MRS medium supplemented with 1% MSG at an initial pH of 5.6, 6.7 and 7 to mimic pH in the proximal and distal colon. The ability to produce GABA under simulated pH conditions is advantageous as the GABA uptake of epithelial colon cells from the apical site is pH dependent. In the study of Thwaites et al., the highest uptake could be measured within a pH <5.6. At pH 8 the lowest GABA uptake was detected (Thwaites et al. 2000).

The total amount of DL-GLU at the baseline (0) was determined to calculate the conversion rate out of 10 g/L MSG (available as deionized DL-GLU; M=147.13 g/mol)) after production of GABA molecules (M=103.1 g/mol). Produced GABA molecules are given in mM and conversion rate in %. The quantitative determination of GABA was done according to Example 1.

DSM34915 was tested under different colon related pH levels as described. It has been impressively demonstrated that DSM34915 can produce GABA under both proximal and distal intestinal conditions. The strain produces 71.43 mM, 73.78 and 19.41 mM of GABA under pH conditions of 5.6, 6.7 and 7. The highest conversion rate was achieved at a pH of 6.7. Here, a 99.90% turnover rate could be shown.

**Table 3: Amounts of GABA produced by DSM34915 in a small cultivation in MRS medium supplemented with 1 wt.-% of monosodium glutamate, containing DL-Glu in an amount of 10.7-10.8 g/L at pH 5.6, 6.7 and 7.0. Total GABA values in mM and normalized values related to the final OD measured at 660 nm are depicted, as well the conversion rate from the initial DL-Glu into GABA.**

| Total GABA [mM] | GABA [mM]/ OD | Conversion rate [%] | Total GABA [mM] | GABA [mM]/ OD | Conversion rate [%] | Total GABA [mM] | GABA [mM]/O D | Conversion rate [%] |
|---|---|---|---|---|---|---|---|---|
| pH 5.6 | pH 5.6 | | pH 6.7 | pH 6.7 | | pH 7 | pH 7 | |
| 71.43 | 19.79 | 98.36 | 73.78 | 21.45 | 99.90 | 19.41 | 6.52 | 26.33 |

### Example 3: Survival under simulated gastric, small, and large intestinal conditions

The survival test of the strain *Levilactobacillus levis* DSM 34915 under simulated gastric, small, and large intestinal conditions was conducted to evaluate the ability of these bacteria to survive and colonize the human gastrointestinal tract. The human gut is a complex environment with different pH levels, digestive enzymes, and microbial populations in different regions. Therefore, it is important to test the viability and stability of probiotic bacteria under these conditions to ensure their potential health benefits. The resistance to artificial gastric and intestinal fluids provides information on the ability of the strain to survive the acidic conditions of the stomach, the bile salts and enzymes in the small intestine, and the anaerobic conditions in simulated media composition of the large intestine and the colon. The survival of the probiotic GABA-producing bacterium DSM 34915 was simulated in an *in-vitro* test by using artificial gastric fluid of pH 2 and intestinal fluid of pH 8, as well as under simulated-colonic-environment medium (SCEM) at pH 7. Simulated gastric fluid (SGF: 125 mmol/L NaCl, 7 mmol/L KCI, 45 mmol/L NaHCOs, 3g/L pepsin, pH adjusted with HCl to 2) and intestinal fluid (SIF: 0.1 % pancreatin, 0.15 % oxgall bile salts in 1x PBS adjusted to pH 8 with 5 mol/L NaOH) were prepared as described by Fernandez (Fernández, Boris, and Barbés 2003). Simulated gastric digestion was tested essentially as described in Zarate et al. 2000 with some modifications. As described in Example 1 and 2, a single colony of tested strain was inoculated in MRS medium supplemented with 0.05% L-cysteine. The culture was incubated at 37°C anaerobically for 24 h. After washing in sterile 1 × PBS buffer (8 g/L NaCl, 1.44 g/L Na₂PO4, 0.24 g/L KH₂PO₄, 0.2 g/L KCI, pH 7.1) and centrifugation, the cell pellet of >1.0E+09 CFU/ml (appr. OD₆₆₀ₙₘ 10) were added to 5 ml of artificial gastric juice. The bacterial suspensions were incubated at 37°C with agitation (200 rev/min) to simulated peristalsis for 120 min. Aliquots were taken for the enumeration of viable at 0 and 120 min. The effect of intestinal digestion was determined by suspending the cells pellet in intestinal fluid at pH 8 after removing gastric fluid by centrifugation for 5 min and 5000 rpm and incubation for further 120 min under peristaltic conditions. The suspension was incubated as above and samples for total viable counts were taken at 0 (120) and 120 (240) min. After the simulated intestinal fluid, the cell pellet was incubated in SCEM medium (6.25 g/L Bacto tryptone, 2.6 g/L D-glucose, 0.88 g/L NaCl, 0.43g/L KH₂PO₄, 1.7 g/L NaHCOs, 2.7 g/L KHCOs, and 4.0 g/L bile salts) (Müsken et al. 2008) to monitor the survival under colonic environment for further 20 h. As well here, viable count in CFU/ml were done on MRS plates for each approach and plates were incubated at 37°C anaerobically for at least two days. As a control, each strain was incubated in 1 × PBS at neutral pH and was prepared as described above without resuspending in gastric and intestinal fluid. The experiment was performed in biological duplicates and counts in technical duplicates.

In Table 4 the CFU counts of DSM34915 after simulated gastrointestinal conditions (GIT: 2 h incubation with SGF and 2h in SIF) and neutral conditions are shown. The initial start concentration was set to <1,0E+09 CFU/ml. 86 % of initial counted cells survive 4 h the control treatment (PBS, centrifugation, resuspension) whereas DSM34915 survives the GIT treatment differently. 92% of initially used DSM34915 cells survive the GIT treatment. An additional incubation in SCEM medium demonstrates that the strain can also survive under minimal salt, nutrients and bile salts conditions. This shows that DSM34915 is highly resistance to pH 2.5 conditions and can deal with enzymatic enzymes and bile salts from the gastrointestinal tract. Figure 2 demonstrates that DSM34915 is a very good strain for overcoming the low pH in the stomach and enzymatic stress in the intestine.

**Table 4: Survival rates measured in CFU/ml and calculated in % of the strains with gastrointestinal treatment and under neutral conditions are shown. Mean values of biological and technical duplicates are given.**

| | | Survival [CFU/ml] | | | | Survival rate [%] | |
|---|---|---|---|---|---|---|---|
| Strain | Condition | 0h | 2h (GIT: SGF pH 2.5) | 4h (GIT: SIF pH 8) | 20h (SCEM pH 7) | 4h | 20 h |
| DSM34915 | control (PBS pH neutral) | 3.96E+09 | 3.77E+09 | 3.39E+09 | 1.29E+09 | 86 | 33 |
| DSM34915 | GIT (SGF+SIF) | 3.96E+09 | 3.81E+09 | 3.64E+09 | 1.71E+09 | 92 | 43 |

### Example 4: Formation of GABA from different substrate sources

The conversion of GABA from components of the MRS medium and the substrate monosodium glutamate (MSG) was shown in the previous Examples. In Example 4 it is shown that the strain DSM 34915 can produce GABA not only in MRS medium with MSG but also from other chemical and natural sources with a high content of glutamic acid. This approach offers a sustainable alternative to chemical synthesis and extraction from natural sources. Here, as additional substrate Alanyl-L-glutamine (Ala-Gln, CAS: 39537-23-0), vegan BIO Spirulina extract (seaweed, dried powder from BIONUTRA; Batch No.: SRORGLXZ221001F), soyprotein-acidic hydrolysated (Sigma-Aldrich; S1674) and a self-prepared DSM32315 hydrolysate were used, and GABA production was measured after 24 h of cultivation. Optimal conditions for GABA production were selected from the experiments shown in Example 1 and 2. MRS-medium was used as described in the previous experiments. 10 g/L Alanyl-L-glutamine (Ala-Gln) from Sigma-Aldrich, 10 g/L vegan BIO Spirulina extract powder from BIONUTRA, 10 g/L acidic hydrolysated soyprotein purchased from Sigma-Aldrich and 10 g/L self-prepared bacterial hydrolysate of the probiotic *Bacillus subtilis* DSM32315 strain. All substrates were diluted in MRS medium, autoclaved and used as culture medium for GABA production by our test strains. The glutamic acid (DL-GLU) content of spirulina extract is 9.715 g/kg. The Bacillus hydrolysate was self-prepared from a cultivation step with the DSM32315 strain. The strain was cultivated in 2x 200 ml tryptic soy medium (Merck, Germany) additionally added with 7 g/L acidic hydrolyzed soy protein (Sigma-Aldrich; S1674) for 16 h at 37°C with 150 rpm. The biomass and culture broth were autoclaved for 15 min at 121°C and the complete material freeze dried for 48 h. The freeze-dried hydrolysate was used as substrate for the GABA production. GABA production was tested in combination with DSM34915. Therefore, a single colony was used as inoculum for pre-cultivation of the strain in biological duplicates. 10 ml MRS medium supplemented with 0.05% Cys-HCL was inoculated and grown for 24 h without agitation at 37°C. The main cultures were prepared with the different substrates in a concentration of 10 g/L beforehand and inoculated with preculture material to a final OD660nm of 0.2. The approaches were cultivated for 24 h at 37°C without agitation in biological duplicates. The quantitative determinations of DL-GLU and GABA were done according to Example 1.

DSM34915 can use different substrate sources for the formation of GABA. The amount of glutamic acid in the different substrates is crucial for the conversion. The more substrate is available or made available, the more GABA can be converted. DSM34915 can even produce higher amounts of GABA with alternated substrate sources. Moreover, almost all substrate is used which is shown by the high conversion rates regarding DL-glutamic acid to GABA.

**Table 5: Amounts of GABA produced by DSM34915 in combination with different substrate sources in combination with MRS. Total GABA values are normalized and given in mM. The conversion rate is calculated based on the available precursor source (DL_GLU molecules) in the condition without strain inoculum. Mean values are given.**

| Substrate source | GABA [mM] | Conversion [%] |
|---|---|---|
| MRS | 5.60 | 102 |
| MRS + 1 wt.-% Ala-Gln | 7.38 | 94 |
| MRS + 1 wt.-% Spirulina | 6.30 | 98 |
| 1 wt.-% acidic-hydrolyzed soyprotein | 10.01 | 88 |
| 1 wt.-% DSM32315 hydrolysate | 6.61 | 98 |

### Example 5: Formation of GABA in comparison to benchmark strains

The formation of GABA in dependency of different initial pH levels was tested in reference to two already in literature described reference strains of the species *Levilactobacillus brevis (Banerjee et al. 2021; Gong, Ren, and Xu 2019*).The pH-dependent GABA production is of high interest as different pH levels can be found at different sites of the human gastrointestinal tract. It was shown before that DSM34915 does survive low pH and different gastrointestinal enzymes. Besides that, it would be important for the strain to also produce GABA at a wide range of pH values, as triggering the GABA uptake by enterocytes works best at acidic pH, as enterocytes have the preference to take up high amounts of GABA at a pH <5.6 (Thwaites et al. 2000). For assessing the ability in comparison to state of the art strains, DSM34915, ATCC367 and DSM20054 were tested in MRS medium as described above supplemented with 1 % monosodium glutamate (10 g/L MSG, >99%, Sigma-Aldrich G1626) and adjusted to different initial pH levels using HCL and NaOH. Prior to the assessment of GABA production, the strain was cultivated on MRS-agar (VWR; 1.5% agar-agar-BioScience, granulated, Roth (1347.2)) for two days at 30°C under anaerobic conditions (90% N₂and 10% CO₂; Oxoid^{™} AnaeroGen^{™} 2.5 L packs - Thermo Scientific, AN0025A). One colony each was further cultivated in 10 ml MRS medium supplemented with 0.05% L-cysteine-HCL as mentioned in Example 1. Subsequently, the strains were inoculated at a concentration of approximately 5×10⁷ CFU/ml (final OD₆₆₀ₙₘ of 0.25) in a Woulter-Deutz small cultivation system containing 8 ml of prepared MRS-medium supplemented with 1% (w/v; 59 mM) MSG at pH 4.5, 5, 5.5, 5.7, 6, 6.7, 7 and 7.2. Bacterial cultures were incubated for 24 h at 37°C under anaerobic conditions (Anaerobic jar 2.5 L; Oxoid^{™} AnaeroGen^{™} Compact packs - Thermo Scientific, AN0020D). Samples were collected at baseline (0) and after 24 h of cultivation and cell-free supernatant were analyzed using liquid chromatographic separation of amino acids as described in Example 1. However, the concentration of L-glutamate is given as DL-glutamic acid as both are two forms of the same amino acid. L-glutamate is the ionized form of L-glutamic acid. In aqueous solution, L-glutamic acid is rapidly deprotonated to form L-glutamate. That is why L-glutamate is measured as L-glutamic acid analytically and used interchangeably. The amounts of GABA produced within 24 h of cultivation are compared among the three strains.

As it is shown in Figure 4, DSM34915 is able to produce high amounts of GABA under different pH levels. The highest GABA production (5.575 g/L) from 1% of MSG could be measured with the DSM34915 at an initial pH level of 5.7 within the 24 h-cultivation. ATCC367 produces only 4.675 g/L GABA under similar conditions. The strain can produce higher amounts under pH 6 but never reaches the concentration measured by DSM34915. The reference strain DSM20054 is able to produce GABA but the highest amount could only be measured at pH 5.7 and is not even half of what the DSM34915 can do. Moreover, the experiment shows that the strain DSM34915 can produce more than 5 g/L at a broad pH ranging from 4.5-6.7. Even at an initial pH of 7 or 7.2 the strain produces 3.8 and 2.45 g/L GABA, respectively. Neither ATCC367 nor DSM20054 can produce such high GABA amounts under the selected conditions.

To check whether the strain DSM34915 can produce even higher amounts of GABA with higher amounts of synbiotic substrate, the amount of MSG supplement was increased to 2.5 wt.-% or 5 wt.-%, respectively. The experiment was performed as described above but with such higher amounts of MSG in a 48 h cultivation showing an increase in GABA production over time for the strain DSM34915. The so far highest GABA concentration of 20.95 g/L was produced by the strain DSM34915 at an initial pH of 4.5 with 5 % MSG (Tab. 6).

**Table 6: Amounts of GABA produced by DSM34915 in a small cultivation in MRS medium supplemented with 2.5 or 5 wt.-% of monosodium glutamate (MSG) at pH 4.5-7.2. Total GABA values in g/L and the conversion rate from MSG to GABA are provided.**

| Concentration | Initial pH condition | GABA [g/L] | GABA [mM] | Conversion rates [%] |
|---|---|---|---|---|
| 2.5 wt.-% MSG | 4.5 | 10.999 | 106.688 | 72 |
| 2.5 wt.-% MSG | 5 | 11.859 | 115.024 | 78 |
| 2.5 wt.-% MSG | 5.5 | 12.349 | 119.777 | 81 |
| 2.5 wt.-% MSG | 5.7 | 12.362 | 119.903 | 81 |
| 2.5 wt.-% MSG | 6 | 11.8705 | 115.136 | 78 |
| 2.5 wt.-% MSG | 6.7 | 11.8255 | 114.699 | 78 |
| 2.5 wt.-% MSG | 7 | 10.222 | 99.146 | 67 |
| 2.5 wt.-% MSG | 7.2 | 5.846 | 56.702 | 38 |
| 5 wt.-% MSG | 4.5 | 20.946 | 203.162 | 69 |
| 5 wt.-% MSG | 5 | 19.537 | 189.496 | 64 |
| 5 wt.-% MSG | 5.5 | 18.037 | 174.947 | 59 |
| 5 wt.-% MSG | 5.7 | 17.637 | 171.067 | 58 |
| 5 wt.-% MSG | 6 | 17.937 | 173.977 | 59 |
| 5 wt.-% MSG | 6.7 | 16.537 | 160.398 | 54 |
| 5 wt.-% MSG | 7 | 6.08 | 58.972 | 20 |
| 5 wt.-% MSG | 7.2 | 3.206 | 31.096 | 11 |

### References

Aggarwal, S., V. Ahuja, and J. Paul. 2018. 'Dysregulation of GABAergic Signalling Contributes in the Pathogenesis of Diarrhea-predominant Irritable Bowel Syndrome', J Neurogastroenterol Motil, 24: 422-30.
Banerjee, S., M. Poore, S. Gerdes, D. Nedveck, L. Lauridsen, H. T. Kristensen, H. M. Jensen, P. M. Byrd, A. C. Ouwehand, E. Patterson, and W. Morovic. 2021. 'Transcriptomics reveal different metabolic strategies for acid resistance and gamma-aminobutyric acid (GABA) production in select Levilactobacillus brevis strains', Microb Cell Fact, 20: 173.
Chen, M., G. Ruan, L. Chen, S. Ying, G. Li, F. Xu, Z. Xiao, Y. Tian, L. Lv, Y. Ping, Y. Cheng, and Y. Wei. 2022. 'Neurotransmitter and Intestinal Interactions: Focus on the Microbiota-Gut-Brain Axis in Irritable Bowel Syndrome', Front Endocrinol (Lausanne), 13: 817100.
Fernández, M.F., S. Boris, and C. Barbés. 2003. 'Probiotic properties of human lactobacilli strains to be used in the gastrointestinal tract', Journal of Applied Microbiology, 94: 449-55.
Gong, L., C. Ren, and Y. Xu. 2019. 'Deciphering the crucial roles of transcriptional regulator GadR on gamma-aminobutyric acid production and acid resistance in Lactobacillus brevis', Microb Cell Fact, 18: 108.
Laroute, V., C. Beaufrand, P. Gomes, S. Nouaille, V. Tondereau, M. L. Daveran-Mingot, V. Theodorou, H. Eutamene, M. Mercier-Bonin, and M. Cocaign-Bousquet. 2022. 'Lactococcus lactis NCDO2118 exerts visceral antinociceptive properties in rat via GABA production in the gastro-intestinal tract', Elife, 11.
Lyu, C., W. Zhao, C. Peng, S. Hu, H. Fang, Y. Hua, S. Yao, J. Huang, and L. Mei. 2018. 'Exploring the contributions of two glutamate decarboxylase isozymes in Lactobacillus brevis to acid resistance and gamma-aminobutyric acid production', Microb Cell Fact, 17: 180.
Monteagudo-Mera, A., V. Fanti, C. Rodriguez-Sobstel, G. Gibson, A. Wijeyesekera, K. A. Karatzas, and B. Chakrabarti. 2023. 'Gamma aminobutyric acid production by commercially available probiotic strains', J Appl Microbiol, 134.
Müsken, Anne, Martina Bielaszewska, Lilo Greune, Christian H. Schweppe, Johannes Müthing, Herbert Schmidt, M. Alexander Schmidt, Helge Karch, and Wenlan Zhang. 2008. 'Anaerobic Conditions Promote Expression of Sfp Fimbriae and Adherence of Sorbitol-Fermenting Enterohemorrhagic <i>Escherichia coli</i> O157:NM to Human Intestinal Epithelial Cells', Applied and Environmental Microbiology, 74: 1087-93.
Thwaites, D. T., L. Basterfield, P. M. McCleave, S. M. Carter, and N. L. Simmons. 2000. 'Gamma-Aminobutyric acid (GABA) transport across human intestinal epithelial (Caco-2) cell monolayers', BrJ Pharmacol, 129: 457-64.

## Claims

1. Composition comprising a microorganism or microbial preparation selected from the following group:
a) *Levilactobacillus brevis* DSM 34915;
b) a mutant of the strain *Levilactobacillus brevis* DSM 34915 with a sequence identity to the genomic DNA of the strain *Levilactobacillus brevis* DSM 34915 of at least 95 %, preferably of at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be cell-free or may contain living or dead cells or cell debris of such a microorganism or any combination thereof.

2. Pharmaceutical composition containing a pharmaceutically acceptable carrier and at least one microorganism or microbial preparation selected from the following group:
a) *Levilactobacillus brevis* DSM 34915;
b) a mutant of the strain *Levilactobacillus brevis* DSM 34915 with a sequence identity to the genomic DNA of the strain *Levilactobacillus brevis* DSM 34915 of at least 95 %, preferably of at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to a) or b), wherein the preparation may be cell-free or may contain living or dead cells or cell debris of such a microorganism or any combination thereof.

3. Composition according to claim 1 or 2, wherein the composition comprises living cells of *Levilactobacillus brevis* DSM 34915 or of a mutant thereof, preferably in an amount of from 1×10² to 1×10¹² CFU, more preferably in an amount of 1×10³ to 1×10¹⁰ CFU, above all in an amount of 1×10⁴ to 1×10⁹ CFU.

4. Composition according to any of the preceding claims, wherein the microorganism or mutant thereof possess at least one, preferably at least two or three, more preferably all, of the following characteristics:
- Ability to grow in presence of 2 mM of bile, preferably in presence of 4 mM of bile;
- Ability to survive exposure to a pH of 2.5 for at least 2 hours with a survival rate of preferably at least 50 %, more preferably at least 70 %;
- Ability to survive the exposure to 0.1 mM pepsin at 37°C for at least 4 hours with a survival rate of preferably at least 50 %, more preferably at least 70 %;
- Ability to convert at least 95 %, preferably at least 98 %, more preferably at least 99 %, of the L-glutamate contained in an MRS medium supplemented with 1 wt.-% of monosodium glutamate into GABA at an initial pH value of 6.7 within 48 hours in a batch cultivation.

5. Composition according to any of the preceding claims, wherein the composition contains glutamic acid or a salt thereof or at least one source for glutamic acid or of a salt thereof, wherein the source of glutamic acid or salt thereof is preferably selected from peptides, protein hydrolysates, Spirulina powder, bacterial extracts, gamma-polyglutamate and Natto powder.

6. Composition according to any of the preceding claims, wherein the composition contains at least one, preferably at least two or three, further substances selected from carriers, further probiotics, prebiotics, enzymes, vitamins, minerals, amino acids, peptides, plant extracts and algal extracts.

7. Composition according to any of the preceding claims, wherein the composition is a composition for oral administration, preferably selected from food and feed compositions, food and feed supplements, food and feed additives, dietary supplements, nutraceuticals and functional foods and preferably contains at least one further food or feed additive, in particular selected from proteins, carbohydrates, fats, feed concentrates, silage, mashfeed, immune modulators, milk replacers, acid-based products and/or medicines, in particular antibiotics.

8. Composition according to any of claims 1 to 6, wherein the composition is a cosmetical composition, preferably for topical application, and preferably contains at least one further additive selected from surfactants, emulsifiers, phospholipids, sphingoid bases, free fatty acids, protein hydrolysates, polymers, light stabilizers, anti-perspirants, deodorants, cosmetic oils, emollients, antioxidants, preservatives, thickeners, viscosity regulators, stabilizers, hydrotropes, solids, fillers, film formers, conditioners, insect repellents, self-tanning agents, odor absorbers, solvents, perfumes and dyes.

9. Composition according to any of the preceding claims for use in the treatment or prevention of mental, mood and/or sleep disorders or diseases, in particular of pain, anxiety, stress and/or loss of motor control and/or of disorders or diseases of the gastrointestinal tract, kidney, skin, brain or vascular system, in particular hypertension or high heart rate.

10. Composition according to any of the preceding claims, wherein the composition is a coated composition and/or contained in a capsule, wherein the coated composition comprises preferably an enteric coating, in particular comprising methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate or zein or combinations thereof and wherein the capsule is preferably a bile-resistant capsule.

11. Product containing a microorganism or microbial preparation selected from the following group:
a) *Levilactobacillus brevis* DSM 34915;
b) a mutant of the strain *Levilactobacillus brevis* DSM 34915 with a sequence identity to the genomic DNA of the strain *Levilactobacillus brevis* DSM 34915 of at least 95 %, preferably of at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to (a) or (b) wherein the preparation may be cell-free or may contain living or dead cells or cell debris of such a microorganism or any combination thereof;
wherein the product is preferably a personal care article, wherein the personal care article is preferably a disposable absorbent article, in particular selected from sanitary napkins, panty liners, tampons, diapers, incontinent pads, breast pads, perspiration pads, human or animal waste management devices and interlabial pads.

12. Microorganism or microbial preparation selected from the following group:
a) *Levilactobacillus brevis* DSM 34915;
b) a mutant of the strain *Levilactobacillus brevis* DSM 34915 with a sequence identity to the genomic DNA of the strain *Levilactobacillus brevis* DSM 34915 of at least 95 %, preferably at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to (a) or (b) wherein the preparation may be cell-free or may contain living or dead cells or cell debris of such a microorganism or any combination thereof.

13. Use of a microorganism or microbial preparation according to claim 12 or of a composition according to any of claims 1 to 10 in the manufacture of a pharmaceutical composition for treating or preventing mental, mood and/or sleep disorders or diseases, in particular pain, anxiety, stress and/or loss of motor control, and/or disorders or diseases of the gastrointestinal tract, kidney, skin, brain or vascular system, in particular hypertension or high heart rate.

14. Non-medical use of a microorganism or microbial preparation according to claim 12 or of a composition according to any of claims 1 or 2 to 10 or of a product according to claim 11 for alleviating or preventing mental, mood and/or sleep disorders or disbalances, in particular anxiety and/or stress.

15. Method of producing GABA, wherein a microorganism according to claim 12 is employed, wherein the microorganism is preferably cultivated in a medium which contains glutamic acid or a salt thereof or at least one source for glutamic acid or of a salt thereof, wherein the source of glutamic acid or salt thereof is preferably selected from peptides, protein hydrolysates, Spirulina powder, bacterial extracts, gamma-polyglutamate and Natto powder.
